# EUROPÄISCHE PATENTSCHRIFT

(11) **EP 0 815 837 B1**
(45) Veröffentlichungstag und Bekanntmachung des Hinweises auf die Patenterteilung: **04.10.2001**
(21) Anmeldenummer: 97110498.9
(22) Anmeldetag: 26.06.1997
(51) Int. Cl.: A61K 7/48, A61K 7/00

(54) **Verwendung von Dialkylethern**
Use of dialkylethers
Utilisation de dialkyléthers

(30) Priorität: 04.07.1996 DE 19626905
(43) Veröffentlichungstag der Anmeldung: 07.01.1998
(73) Patentinhaber: Cognis Deutschland GmbH, 40589 Düsseldorf (DE)
(72) Erfinder: Ansmann, Achim, Dr. Dipl.-Chem., 40699 Erkrath (DE)

(56) Entgegenhaltungen:
- EP-A- 0 264 844
- DE-A- 4 243 272

## Beschreibung

### Gebiet der Erfindung

Die Erfindung betrifft die Verwendung von Dialkylethern als Ölkörper für klare und homogene Zubereitungen mit Siliconölen.

### Stand der Technik

Siliconöle werden in der Haut- und Haarkosmetik als Additive zur Beeinflussung von Griff und Glanz eingesetzt. Eine Übersicht hierzu findet sich beispielsweise von K.Schnurrbusch in **Seifen-Fette-Öle-Wachse 100**, **173, 1974).** Aus der deutschen Offenlegungsschrift **DE-A1 4243272** (Henkel) ist in diesem Zusammenhang ein Verfahren zur Herstellung von O/W-Emulsionen nach der Phaseninversionstemperatur-Methode bekannt, bei dem man Siliconöle zusammen mit ausgewählten Emulgatoren und nichtionischen Verbindungen wie z.B. Dialkylethern, in Kontakt bringt. Ein besonderes Problem besteht jedoch darin, Formulierungen herzustellen, die einerseits einen möglichst hohen Anteil an Siliconölen enthalten und die gleichzeitig über einen längeren Zeitraum klar und homogen bleiben, d.h. in denen das Siliconöl keine Tendenz zeigt, allmählich auszutrüben. Die Aufgabe der Erfindung hat somit darin bestanden, Ölkörper zur Verfügung zu stellen, die frei von den geschilderten Nachteilen sind.

### Beschreibung der Erfindung

Gegenstand der Erfindung ist die Verwendung von Dialkylethern als Ölkörper für klare und homogene Zubereitungen mit Siliconölen.

Überraschenderweise wurde gefunden, daß unter der Vielzahl in Betracht kommender Ölkörper ausgerechnet Dialkylether die Formulierung trübungsfreier und lagerstabiler Formulierungen mit Siliconölen zulassen. Die Erfindung schließt die Erkenntnis ein, daß auf diesem Wege sowohl die Einarbeitung ganz beträchtlicher Mengen Dialkylether als auch Siliconöl möglich ist.

### Dialkylether

Dialkylether stellen bekannte kosmetische Ölkörper dar, die man beispielsweise durch saure Kondensation von geeigneten primären Alkoholen erhält. Vorzugsweise werden Dialkylether eingesetzt, die der Formel **(I)** folgen,

**R**^{**1**}**-O-R**^{**2**} **(I)**

in der R¹ und R² unabhängig voneinander für lineare oder verzweigte Alkylreste mit 6 bis 22 Kohlenstoffatomen stehen. Typische Beispiele sind Di-n-octylether, Di-i-octylether, Di-i-laurylether, Di-cetylether, Di-n-stearylether und Di-i-stearylether.

### Siliconöle

Geeignete Siliconöle sind beispielsweise Dimethylpolysiloxane, Methylphenylpolysiloxane, cyclische Silicone sowie amino-, fettsäure-, alkohol-, polyether-, epoxy- fluor-, glucosid- und/oder alkylmodifizierte Siliconverbindungen, die bei Raumtemperatur sowohl flüssig als auch harzförmig vorliegen können. Üblicherweise setzt man die Dialkylether und die Siliconöle im Gewichtsverhältnis 5 : 1 bis 1 : 5 und insbesondere 1 2 bis 2 : 1 ein. In der Regel enthalten die Zubereitungen 20 bis 75, vorzugsweise 25 bis 50 und insbesondere 30 bis 40 Gew.-% Siliconöle. Vorzugsweise sind die Zubereitungen wasserfrei und enthalten nur die Siliconöle und die Dialkylether.

### Gewerbliche Anwendbarkeit

Im Sinne der erfindungsgemäßen Verwendung lassen sich kosmetische bzw. pharmazeutische Zubereitungen mit Siliconölen für die Anwendung auf Haut und Haaren herstellen, die auch bei längerer Lagerung nicht austrüben und die Einarbeitung auch größerer Mengen sowohl Dialkylether als auch Silicone gestatten.

Die Mittel können in untergeordneten Mengen mit den anderen Inhaltsstoffen kompatible Tenside enthalten. Typische Beispiele sind Fettalkoholpolyglycolethersulfate, Monoglyceridsulfate, Mono- und/ oder Dialkylsulfosuccinate, Fettsäureisethionate, Fettsäuresarcosinate, Fettsäuretauride, Ethercarbonsäuren, Alkyloligoglucoside, Fettsäureglucamide, Acylglutamate, Alkylphosphate, Alkylamidobetaine und/oder Proteinfettsäurekondensate.

Als weitere Hilfs- und Zusatzstoffe können Emulgatoren, Überfettungsmittel, Stabilisatoren, Wachse, Konsistenzgeber, Verdickungsmittel, Kationpolymere, biogene Wirkstoffe, Antischuppenmittel, Filmbildner, Konservierungsmittel, Hydrotrope, Solubilisatoren, UV-Adsorber, Farb- und Duftstoffe enthalten sein.

Als **Emulgatoren** kommen beispielsweise nichtionogene Tenside aus mindestens einer der folgenden Gruppen in Frage:
(b1) Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe;
(b2) C_{12/18}-Fettsäuremono- und -diester von Anlagerungsprodukten von 1 bis 30 Mol Ethylenoxid an Glycerin;
(b3) Glycerinmono- und -diester und Sorbitanmono- und -diester von gesättigten und ungesättigten Fettsäuren mit 6 bis 22 Kohlenstoffatomen und deren Ethylenoxidanlagerungsprodukte;
(b4) Alkylmono- und -oligoglycoside mit 8 bis 22 Kohlenstoffatomen im Alkylrest und deren ethoxylierte Analoga;
(b5) Anlagerungsprodukte von 15 bis 60 Mol Ethylenoxid an Ricinusöl und/oder gehärtetes Ricinusöl;
(b6) Polyol- und insbesondere Polyglycerinester wie z.B. Polyglycerinpolyricinoleat oder Polyglycerinpoly-12-hydroxystearat. Ebenfalls geeignet sind Gemische von Verbindungen aus mehreren dieser Substanzklassen;
(b7) Anlagerungsprodukte von 2 bis 15 Mol Ethylenoxid an Ricinusöl und/oder gehärtetes Ricinusöl;
(b8) Partialester auf Basis linearer, verzweigter, ungesättigter bzw. gesättigter C_{12/22}-Fettsäuren, Ricinolsäure sowie 12-Hydroxystearinsäure und Glycerin, Polyglycerin, Pentaerythrit, Dipentaerythrit, Zuckeralkohole (z.B. Sorbit) sowie Polyglucoside (z.B. Cellulose);
(b9) Trialkylphosphate;
(b10) Wollwachsalkohole;
(b11) Polysiloxan-Polyalkyl-Polyether-Copolymere bzw. entsprechende Derivate;
(b12) Mischester aus Pentaerythrit, Fettsäuren, Citronensäure und Fettalkohol gemäß **DE-PS 11 65 574,**
(b13) Polyalkylenglycole sowie
(b14) Hydroxycarbonsäurederivate, wie beispielsweise Citronensäureester oder Acyllactylate.

Die Anlagerungsprodukte von Ethylenoxid und/oder von Propylenoxid an Fettalkohole, Fettsäuren, Alkylphenole, Glycerinmono- und -diester sowie Sorbitanmono- und -diester von Fettsäuren oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind aus **DE-PS 20 24 051** als Rückfettungsmittel für kosmetische Zubereitungen bekannt.

C_{8/18}-Alkylmono- und -oligoglycoside, ihre Herstellung und ihre Verwendung als oberflächenaktive Stoffe sind beispielsweise aus **US 3,839,318, US 3,707,535, US 3,547,828, DE-OS 19 43 689, DE-OS 20 36 472** und **DE-A1 30 01 064** sowie **EP-A 0 077 167** bekannt. Ihre Herstellung erfolgt insbesondere durch Umsetzung von Glucose oder Oligosacchariden mit primären Alkoholen mit 8 bis 18 C-Atomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.

Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethylammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung *Cocamidopropyl Betaine* bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C_{12/18}-Acylsarcosin. Neben den ampholytischen kommen auch quartäre Emulgatoren in Betracht, wobei solche vom Typ der Esterquats, vorzugsweise methylquaternierte Difettsäuretriethanolaminester-Salze, besonders bevorzugt sind. Anionische Tenside, wie sie oben exemplarisch bereits genannt worden sind, kommen grundsätzlich auch als anionische Emulgatoren in Frage.

Als **Überfettungsmittel** können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen. Als **Konsistenzgeber** kommen in erster Linie Fettalkohole mit 12 bis 22 und vorzugsweise 16 bis 18 Kohlenstoffatomen in Betracht. Bevorzugt ist eine Kombination dieser Stoffe mit Alkyloligoglucosiden und/oder Fettsäure-N-methyl-glucamiden gleicher Kettenlänge und/oder Polyglycerinpoly-12-hydroxystearaten. Geeignete **Verdickungsmittel** sind beispielsweise Polysaccharide, insbesondere Xanthan-Gum, Guar-Guar, Agar-Agar, Alginate und Tylosen, Carboxymethylcellulose und Hydroxyethylcellulose, ferner höhermolekulare Polyethylenglycolmono- und -diester von Fettsäuren, Polyacrylate wie beispielsweise vom Salcare®-, Synthalen®- oder Sepigel®-Typ, Polyvinylalkohol und Polyvinylpyrrolidon, Tenside wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

Geeignete **kationische Polymere** sind beispielsweise kationische Cellulosederivate, kationische Stärke, Copolymere von Diallylammoniumsalzen und Acrylamiden, quaternierte VinylpyrrolidonNinylimidazol-Polymere wie z.B. Luviquat® (BASF AG, Ludwigshafen/ FRG), Kondensationsprodukte von Polyglycolen und Aminen, quaternierte Kollagenpolypeptide wie beispielsweise Lauryldimonium hydroxypropyl hydrolyzed collagen (Lamequat®L, Grünau GmbH), quaternierte Weizenpolypeptide, Polyethylenimin, kationische Siliconpolymere wie z.B. Amidomethicone oder Dow Corning, Dow Corning Co./US, Copolymere der Adipinsäure und Dimethylaminohydroxypropyldiethylentrimamin (Cartaretine®, Sandoz/CH), Polyaminopolyamide wie z.B. beschrieben in der **FR-A 22 52 840** sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen wie z.B. Dibrombutan mit Bisdialkylaminen wie z.B. Bis-Dimethylamino-1,3-propan, kationischer Guar-Gum wie z.B. Jaguar® CBS, Jaguar® C-17, Jaguar® C-16 der Celanese/US, quaternierte Ammoniumsalz-Polymere wie z.B. Mirapol® A-15, Mirapol® AD-1, Mirapol® AZ-1 der Miranol/US.

Typische Beispiele für **Fette** sind Glyceride, als **Wachse** kommen u.a. Bienenwachs, Paraffinwachs oder Mikrowachse gegebenenfalls in Kombination mit hydrophilen Wachsen, z.B. Cetylstearylalkohol in Frage. Als **Perlglanzwachse** können insbesondere Mono- und Difettsäureester von Polyalkylenglycolen, Partialglyceride oder Ester von Fettalkoholen mit mehrwertigen Carbonsäuren bzw. Hydroxycarbonsäuren verwendet werden. Als **Stabilisatoren** können Metallsalze von Fettsäuren wie z.B. Magnesium-, Aluminium- und/oder Zinkstearat eingesetzt werden. Unter **biogenen Wirkstoffen** sind beispielsweise Bisabolol, Allantoin, Phytantriol, Panthenol, AHA-Säuren, Pflanzenextrakte und Vitaminkomplexe zu verstehen. Als **Antischuppenmittel** können Climbazol, Octopirox und Zinkpyrethion eingesetzt werden. Gebräuchliche **Filmbildner** sind beispielsweise Chitosan, mikrokristallines Chitosan, quaterniertes Chitosan, Polyvinylpyrrolidon, Vinylpyrrolidon-Vinylacetat-Copolymerisate, Polymere der Acrylsäurereihe, quaternäre Cellulose-Derivate, Kollagen, Hyaluronsäure bzw. deren Salze und ähnliche Verbindungen. Zur Verbesserung des Fließverhaltens können ferner **Hydrotrope** wie beispielsweise Ethanol, Isopropylalkohol, Propylenglycol, Glycerin oder Glucose eingesetzt werden. Als **Konservierungsmittel** eignen sich beispielsweise Phenoxyethanol, Formaldehydlösung, Parabene, Pentandiol oder Sorbinsäure. Als **Farbstoffe** können die für kosmetische Zwecke geeigneten und zugelassenen Substanzen verwendet werden, wie sie beispielsweise in der Publikation **"Kosmetische Färbemittel" der Farbstoffkommission der Deutschen Forschungsgemeinschaft, Verlag Chemie, Weinheim, 1984, S.81-106** zusammengestellt sind. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 90, vorzugsweise 5 bis 40 Gew.-% - bezogen auf die Mittel - betragen. Die Herstellung der Mittel kann durch übliche Kalt- oder Heißprozesse erfolgen; vorzugsweise arbeitet man nach der Phaseninversionstemperatur-Methode.

### Beispiele

Bei 30°C wurden Mischungen bestehend aus 50 Gew.-% Ölkörper und 50 Gew.-% Siliconöl hergestellt und anschließend bei 40°C über 3 Wochen gelagert. Anschließend wurden die Formulierungen visuell beurteilt. Dabei wurden folgende Ölkörper getestet (vgl. Tabelle 1):
- R1): Dicaprylyl ether (Cetiol® OE)
- R2): Decyl oleate (Cetiol® V)
- R3): Octyldodecanol (Eutanol® G)
- R4): Caprylic/capric triglyceride (Myritol® 318)
- R5): Dioctyldodecyl carbonate
- R6): Mandelöl

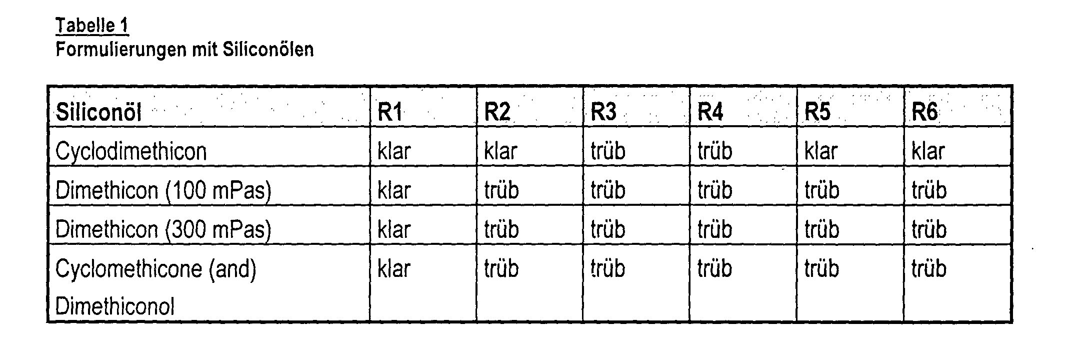

Rezeptur R1 ist erfindungsgemäß, die Rezepturen R2 bis R6 dienen zum Vergleich. Man erkennt, daß man mit den unterschiedlichsten Siliconölen nur dann klare 1:1-Formulierungen erhält, wenn man Dialkylether als Ölkörper einsetzt. In Tabelle 2 sind Rezepturbeispiele enthalten.

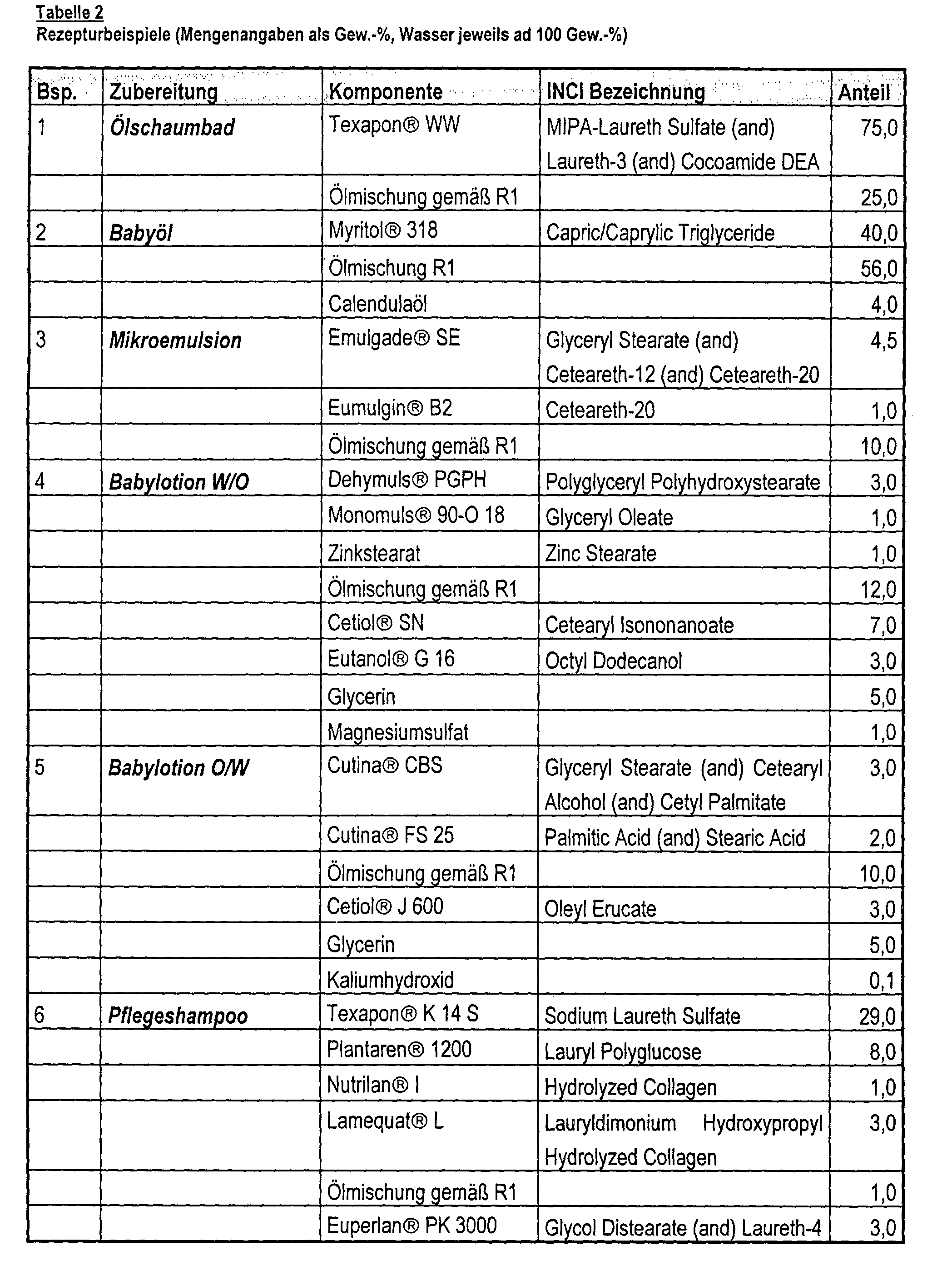

## Patentansprüche

1. Verwendung von Dialkylethern als Ölkörper für klare, homogene Zubereitungen mit Siliconölen.

2. Verwendung nach Anspruch 1, **dadurch gekennzeichnet, daß** man Dialkylether der Formel **(I)** einsetzt,
**R**^{**1**}**-O-R**^{**2**} **(I)**
in der R¹ und R² unabhängig voneinander für lineare oder verzweigte Alkylreste mit 6 bis 22 Kohlenstoffatomen steht.

3. Verwendung nach den Ansprüchen 1 und 2, **dadurch gekennzeichnet, daß** man Siliconöle einsetzt, die ausgewählt sind aus der Gruppe, die gebildet wird von Dimethylpolysiloxanen, Methylphenylpolysiloxanen, cyclischen Siliconen sowie amino-, fettsäure-, alkohol-, polyether-, epoxyfluor-, glucosid- und/oder alkylmodifizierten Siliconen.

4. Verwendung nach den Ansprüchen 1 bis 3, **dadurch gekennzeichnet, daß** man die Dialkylether und die Siliconöle im Gewichtsverhältnis 5 : 1 bis 1 5 einsetzt.

## Claims

1. The use of dialkyl ethers as oil components for clear, homogeneous preparations containing silicone oils.

2. The use claimed in claim 1, **characterized in that** dialkyl ethers corresponding to formula (I):
R¹-O-R² (I)
in which R¹ and R² independently of one another represent linear or branched C₆₋₂₂ alkyl groups,
are used.

3. The use claimed in claims 1 and 2, **characterized in that** silicone oils selected from the group consisting of dimethyl polysiloxanes, methyl phenyl polysiloxanes, cyclic silicones and amino-, fatty acid-, alcohol-, polyether-, epoxy-, fluorine-, glucoside and/or alkyl-modified silicones are used.

4. The use claimed in claims 1 to 3, **characterized in that** the dialkyl ethers and the silicone oils are used in a ratio by weight of 5:1 to 1:5.

## Revendications

1. Utilisation de dialkyléthers comme composés huileux pour des préparations limpides, homogènes avec des huiles de silicone.

2. Utilisation selon la revendication 1,
**caractérisée en ce qu'**
on met en oeuvre des dialkyléthers de formule (I),
R¹-O-R² (I)
dans laquelle R¹ et R² indépendamment l'un ce l'autre représentent des restes alkyle linéaires ou ramifiés ayant de 6 à 22 atomes de carbone.

3. Utilisation selon les revendications 1 et 2,
**caractérisée en ce qu'**
on met en oeuvre des huiles de silicone qui sont choisies dans le groupe qui est formé des diméthylpolysiloxanes, des méthylphénylpolysiloxanes, des silicones cycliques ainsi que des silicones modifiées par un amino, un acide gras, un alcool, un polyéther, un époxy, un fluoro, un glucoside et/ou un alkyle.

4. Utilisation selon les revendications 1 à 3,
**caractérisée en ce qu'**
on met en oeuvre les dialkyléthers et les huiles de silicone dans le rapport en poids allant de 5 : 1 à 1 : 5.
